# EUROPEAN PATENT APPLICATION

(11) **EP 3 918 980 A1**
(43) Date of publication of application: **08.12.2021**
(21) Application number: 20177900.6
(22) Date of filing: 02.06.2020
(51) Int. Cl.: A61B 5/024, A61B 5/0245, A61B 5/00

(54) **METHOD FOR CLASSIFYING PHOTOPLETHYSMOGRAPHY PULSES AND MONITORING OF CARDIAC ARRHYTHMIAS**

(71) Applicant: CSEM Centre Suisse d'Electronique et de Microtechnique SA - Recherche et Développement, 2002 Neuchâtel (CH)
(72) Inventor: Braun, Fabian, 2000 Neuchâtel (CH); Proença, Martin, 1786 Sugiez (CH); Van Zaen, Jérôme, 1007 Lausanne (CH); Renevey, Philippe, 1005 Lausanne (CH); Lemay, Mathieu, 1350 Orbe (CH)
(74) Representative: P&TS SA (AG, Ltd.)

(57) **Abstract**

A method based on pulse wave analysis for monitoring cardiovascular vital signs, comprising: measuring a photoplethysmography (PPG) signal during a measurement time period such as to obtain a time series of PPG pulses; and during said measurement time period, identifying individual PPG pulses in the PPG signal, each PPG pulse corresponding to a PPG pulse cycle. For each PPG pulse, the method uses a pulse-wave analysis technique to determine, within the pulse cycle, at least one of: a time-related feature comprising a time duration and/or a normalized amplitude-related pulse-related feature and/or a SNR-related pulse-related. For each PPG pulse, the method uses a machine learning model in combination with the determined time-related, normalized amplitude-related and SNR-related features for the PPG pulse, to classify each PPG pulse in the preprocessed PPG signal as "normal", "pathological" or "non-physiological" such as to output a time series of pulse classes.

## Description

### Field

The present disclosure concerns a method for classifying photoplethysmography (PPG) pulses. The present disclosure further concerns a method for detecting atrial fibrillation (AF), classifying arrhythmias and monitoring blood pressure, SpO₂ and sleep and other heart rate variability (HRV) derived parameters.

### Description of related art

The monitoring of cardiac arrhythmias through PPG is a growing field of research. Interest in AF is high since it is the most common arrhythmia affecting millions of individuals worldwide.

Known monitoring methods and products based on PPG-dedicated algorithms are configured for distinguishing sinus (normal) rhythm episodes from AF ones. Typically, sinus and AF episodes are separated from each other by estimating electrocardiogram (ECG)-based and PPG-based RR intervals. The standard deviation of RR intervals is low during sinus rhythm or high during AF. The inverse is observed with average values of RR intervals which are high during sinus rhythm or low AF. For instance, A.G. Bonomi, et. al., "Atrial Fibrillation Detection Using a Novel Cardiac Ambulatory Monitor Based on Photo-Plethysmography at the Wrist", Journal of the American Heart Association, Aug 7; 7(15) (2018), the describes AF detection using photo-plethysmography signals measured from a wrist-based wearable device.

There is a need in a method that can further separate AF episodes from other cardiac arrhythmias, such as premature ventricular and atrial contractions, flutters, supraventricular and ventricular tachycardias, as well as cardiac dysfunctions such as left branch block and AV node reentry.

### Summary

During recent AF-related studies on PPG signals performed by the present inventors, the analysis of the recorded data has pointed at the fact that the addition of features based on variation of PPG pulse morphologies might be necessary to achieve an improved performance to distinguish various types of cardiac arrhythmia. For example, it was observed that, for large number of AF epochs, pathological PPG pulse morphologies were recorded. It was hypothesized that AF leads to changes in hemodynamics such as reduced stroke volume (followed by a reduction in systemic blood pressure) and a pooling of venous blood, resulting in such pathological PPG pulse morphology. Moreover, variations of PPG pulse amplitudes and PPG baseline variations were observed on numerous episodes during sinus rhythm as well as during AF. The variations can reflect stroke volume variations caused by short and long recovery episodes (consecutive short and long RR intervals) combined with the compensation of the sympathetic vasoconstriction due to peripheral pressure increase. This phenomenon is expected to modulate PPG signals and create the observed low frequency component. Although false negative and false positive PPG pulse detections (pulse upstrokes) were observed during AF episodes, the PPG-based RR intervals were similar to ECG-based RR-intervals. Recent studies by the present inventors have shown a high accuracy (e.g. a mean absolute error < 10 ms) when comparing PPG-based vs ECG-based RR intervals.

Following the above AF-related studies, the inventors have concluded that a method that can further separate AF episodes from other cardiac arrhythmias should be based on quantifying the morphology of the PPG pulses and on the classification of the PPG pulse in accordance with their morphologies.

The present disclosure concerns a method based on pulse wave analysis for monitoring cardiovascular vital signs, comprising:
measuring a PPG signal during a measurement time period such as to obtain a time series of PPG pulses; and
during said measurement time period, identifying individual PPG pulses in the PPG signal, each PPG pulse corresponding to a pulse cycle;
for each PPG pulse, using a pulse-wave analysis technique to determine, within the pulse cycle, at least one of: a time-related feature comprising a time duration and/or a normalized amplitude-related pulse-related feature and/or a signal-to-noise ratio (SNR)-related pulse-related feature;
wherein, for each PPG pulse, using a machine learning model in combination with the determined time-related normalized amplitude-related and SNR-related features to classify each PPG pulse in the PPG signal as "normal", "pathological" or "non-physiological" such as to output a time series of pulse classes.

In an embodiment, the method comprises a step of training the machine learning model by using expert-labelled data.

The method disclosed herein allows for accurate classification of a measured PPG pulse and cardiac arrhythmia classification. The method disclosed herein allows for detecting atrial fibrillation (AF), classifying arrhythmias and monitoring blood pressure, SpO₂ and sleep and other HRV-derived parameters.

The method disclosed herein can be used for the ambulatory monitoring of abnormal rhythm episodes and continuous monitoring of cardiac arrhythmia. The method can be used in any known PPG-based sensors.

### Brief Description of the Drawings

The invention will be better understood with the aid of the description of an embodiment given by way of example and illustrated by the figures, in which:
Fig. 1a shows a PPG signal measured by a PPG sensor during a time period;
Fig. 1b shows the PPG signal after removing low frequency components;
Fig. 2a shows a step of identifying cardiac contractions (R-wave peaks), cardiac contraction onsets (P-wave onset) and the corresponding cardiac cycle in an ECG signal;
Fig. 2b shows a step of identifying PPG pulse upstrokes, pulse feet and the corresponding pulse cycle in the PPG signal;
Fig. 3 shows a PPG pulse from which time-related features, normalized amplitude-related features and SNR-related features are extracted;
Fig. 4 illustrates schematically a classifying machine learning model inputted with the time-related, normalized amplitude-related and SNR-related features and outputting a classified time series PPG waveform;
Figs. 5a to 5c represents an ECG signal (Fig. 5a) synchronously with the measured PPG signal (Fig. 5b) and corresponding ECG-based and PPG-based RR intervals (Fig. 5c);
Figs. 6a and 6b shows a measured ECG signal with expert classified labels (Fig. 6a) and a PPG signal measured synchronously with the ECG signal (Fig. 6b); and
Fig. 7 shows such classified PPG pulses.

### Detailed Description of possible embodiments

In describing and claiming the present disclosure, the following terminology will be used.

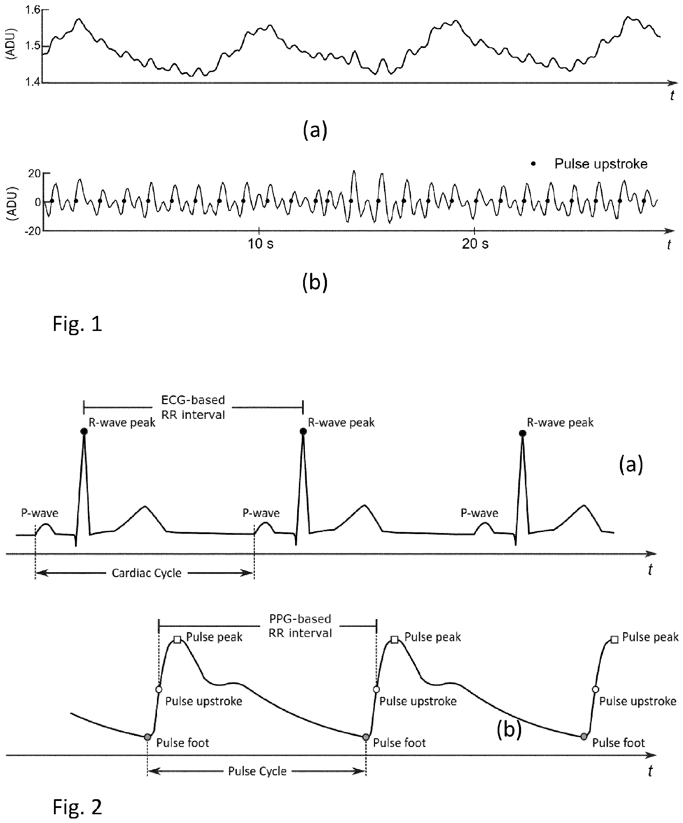

The expression "cardiac contraction" corresponds to the onsets of ventricular contraction of the heart, represented by R-wave peaks in the ECG waveform (black dots in Fig. 2a).

The expression "cardiac cycle" corresponds to the duration between the two successive cardiac contraction onsets (P-wave onsets) of the ECG signal (see Fig. 2a).

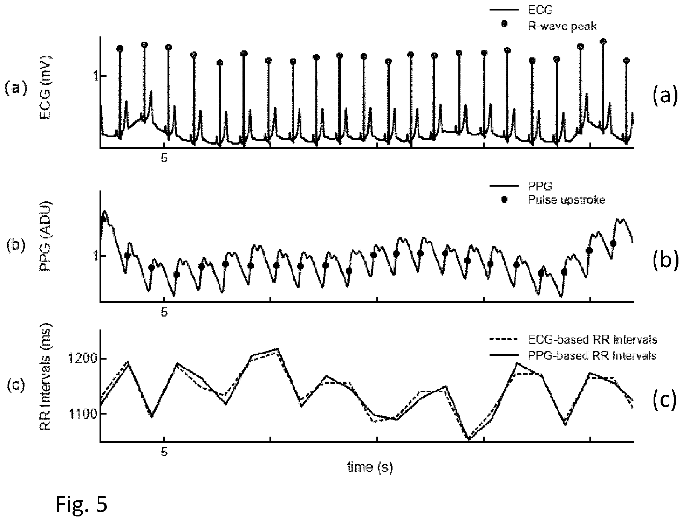

The expression "ECG-based RR intervals" corresponds to the resulting time series representing the time difference between successive cardiac contractions (dashed line in Fig. 5c).

The expression "ECG signal" corresponds to the time series of ECG samples.

The expression "PPG-based RR intervals" corresponds to the resulting time series representing the time difference between successive cardiac contractions (solid line in Fig. 5c). "PPG-based RR intervals" are sometimes also called "PPG-based inter-beat-intervals".

The expression "pulse upstroke" corresponds to the systolic upstrokes in the PPG signals, represented by steep upstrokes (see white dots in Fig. 2b or black dots in Fig. 5b).

The expression "pulse cycle" corresponds to the duration between two successive pulse feet in the PPG signal (see Fig. 2b).

The expression "PPG pulse" corresponds to the PPG signal during a PPG pulse cycle.

The expression "pulse feet" corresponds to the local minima (see gray dots in Fig. 2b) in the PPG signal preceding the pulse upstrokes (see white dots in Fig. 2b) and determine the start of a pulse cycle.

The expression "PPG signal" corresponds to the time series of PPG samples.

The expression "pulse class" corresponds to the resulting classification "normal" (N), "pathological" (P) or "non-physiological" (X) of the PPG pulse provided by a pulse classifier.

The expression "pulse classifier" corresponds to the trained classifying machine learning model.

The expression "time series of pulse classes" corresponds to the output of the pulse classifier which is a time series of PPG classes N, P and X of which each is associated to a PPG pulses.

According to an embodiment, a method based on pulse wave analysis for monitoring cardiovascular vital signs, comprises the steps of:
measuring a PPG signal during a measurement time period such as to obtain a time series of PPG pulses;
during said measurement time period, identifying individual PPG pulses in the PPG signal, wherein each PPG pulse corresponds to the PPG signal during a pulse cycle;
for each PPG pulse, using a pulse-wave analysis technique to determine, within the pulse cycle, at least one of: a time-related feature comprising a time duration, and/or a normalized amplitude-related pulse-related feature, and/or a SNR-related feature; and
for each PPG pulse, using a classifying machine learning model in combination with the determined time-related, normalized amplitude-related and SNR-related features, to classify each PPG pulse in the PPG signal as "normal" (N), "pathological" (P) or "non-physiological" (X) such as to output a time series of pulse classes.

The time series of pulse classes comprises the pulse classes "normal" (N), "pathological" (P) or "non-physiological" (X) attributed to each PPG pulse.

Here, the classification "normal" can include a PPG pulse that has been generated by a normal cardiac contraction (cardiac contraction resulting from sinus node depolarization). The classification "pathological" can include a PPG pulse that has been generated by an abnormal cardiac contraction (cardiac contraction not resulting from sinus node depolarization). The classification "non-physiological" can include a PPG pulse associated to a noisy or unidentified PPG pulse. The classification "non-physiological" can further include PPG pulses having a waveform that is not of purely physiological origin (including PPG pulses with motion artefact, electromagnetic perturbation, low SNR, ambient light perturbation). The classification "non-physiological" can further include PPG pulses with physiological sources that are not directly related to arterial pulsatility (e.g. venous pulsatility, ballistocardiographic effect).

**Fig. 1a** shows a PPG signal comprising PPG pulses and measured by PPG sensor (not shown) located in the vicinity of a vascularized tissue, during a time period. The arrival of a blood pressure pulse in small arteries located in the vicinity of the PPG sensor produces an increase of blood volume that results in an augmentation of the light absorption. Here, the PPG signal is considered to have the same orientation as a blood pressure waveform. In this case the measured PPG signal increases when there is an increase in light absorption.

The PPG sensor typically comprises a light source configured to emit a light signal destined to illuminate a vascularized tissue, and a light detector configured to detect the light signal that has illuminated the tissue. The PPG sensor can comprise transmission-based or reflective-based sensor. The light source may comprise any one of a single or multiple light emitting diodes (LED), single or multiple laser diodes (LD), micro-plasma emitters, thermal sources, organic LEDs or tunable lasers. The light detector can comprise any one of photodiodes, phototransistors or any other light sensitive element or a digital camera.

**Fig. 1b** shows a pre-processed PPG signal, for example after removing low frequency components of the PPG signal. The low frequency components can be removed by removing a baseline wander of the PPG signal. Also shown in Fig. 1b as dots are pulse upstrokes detected from the pre-processed PPG signal. Here, a pulse upstroke corresponds to the timing of a systolic upstroke in the PPG signal.

In one aspect shown in **Fig. 2b****,** the step of identifying pulse upstrokes (white circular dots in Fig. 2b) can be performed by detecting the timing of systolic upstrokes in the PPG signal, in other words by using steepest upwards slope of the PPG signal, for example by using zero-crossings in the second derivative of the PPG signal. Identifying pulse feet can further be performed by detecting local minima preceding the pulse upstrokes in the PPG signal or by any other suitable method. Other fiducial points such as pulse peaks (white squares in Fig. 2b) can be calculated from the PPG signal.

The PPG-based RR intervals can be calculated as the difference between timings of consecutive fiducial points such as pulse upstrokes or pulse feet. Individual PPG pulses (see Fig. 2b) corresponding to the PPG signal during a pulse cycle can then be identified in the PPG signal as the segment of the PPG signal between two successive pulse feet (see gray dots in Fig. 2b), i.e. the local minima preceding the pulse upstrokes (see white dots in Fig. 2b).

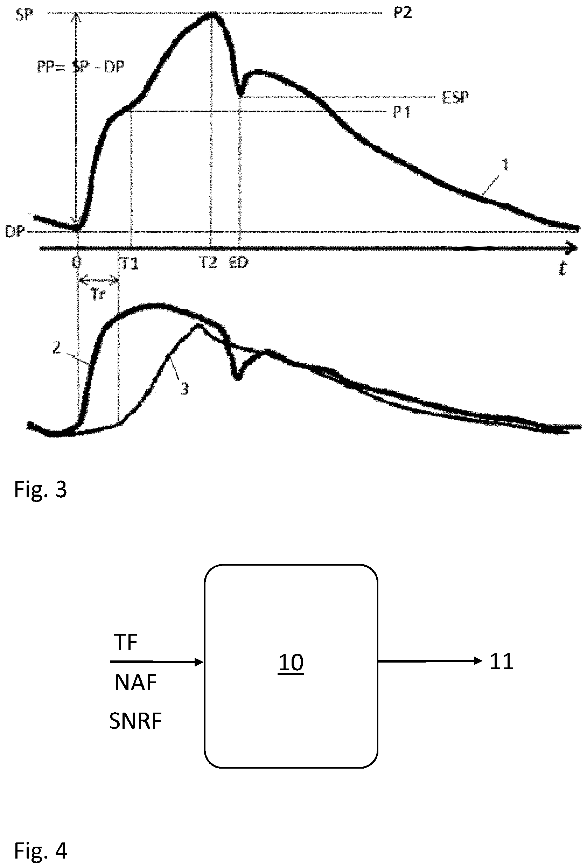

At least one time-related feature (TF) is computed from each PPG pulse of the PPG signal, using a pulse-wave analysis technique (see Fig. 3). The time-related feature can include any time duration within a PPG pulse, the inverse of such a time duration, or any other value computed from such a time duration, e.g. the average of a time duration or its inverse of a plurality of peaks. Examples for such a time-related feature are the time to first peak T1 (duration between the start time of the pulse and its first peak or shoulder of pulse), time to second peak T2 (duration between start time of pulse and second peak or shoulder of pulse), inverse time to first peak 1/T1, inverse of time to second peak 1/T2, time between first and second peak T2-T1, time to reflection Tr (duration between start time of pulse and arrival time of the reflected (backward wave), ejection duration ED (duration between start time of pulse and time of closure of the aortic valve) or heart rate. Other time-related features can also be contemplated.

At least one normalized amplitude-related feature (NAF) is computed from each PPG pulse of the PPG signal, using a pulse-wave analysis technique (Fig. 3). The normalized amplitude-related feature can be any value based on an amplitude value of the PPG signal and normalized by another amplitude-related feature. Normalization can be performed by a ratio of two amplitude-related features.

The normalized amplitude-related feature can include normalized end-systolic pressure nESP=(ESP-DP)/PP calculated by the difference of the absolute end-systolic pressure ESP and the diastolic pressure DP divided or normalized by the pulse pressure PP. The normalized amplitude-related feature can further include a first augmentation index Alx=(P2-P1)/PP calculated by the difference of the pressure amplitude of the second peak P2 and the pressure amplitude of the first peak P1 divided or normalized by the pulse pressure PP. In one aspect, the normalized amplitude-related feature includes a second augmentation index Alp=(P2-DP)/(P1-DP) calculated by the difference of the pressure amplitude of the second peak P2 and the diastolic pressure amplitude DP divided or normalized by the difference of the pressure amplitude of the first peak P1 and the diastolic pressure amplitude DP. In another aspect, the normalized amplitude-related feature includes a normalized ejection area. The normalized ejection area can be calculated as the surface under the PPG pulse for the ejection duration ED, normalized by the area under the PPG pulse for the duration of this pulse. Other normalized amplitude-related features can also be considered.

At least one SNR-related feature (SNRF) is computed for each PPG pulse of the PPG signal. The SNR is computed for example by computing the number of zero-crossings of the first-time derivative of the PPG pulse. The SNR can be computed using any other suitable method.

**Fig. 4** illustrates schematically the classifying machine learning model 10 inputted with the time-related TF, normalized amplitude NAF and signal-to-noise ratio SNRF related features, and determined for each PPG pulse. The classifying machine learning model 10 classifies each PPG pulse in the pre-processed PPG signal as "normal", "pathological" or "non-physiological" and outputs a time series of PPG pulse classes 11. In other words, the classifying machine learning model 10 automatically assigns a class "normal", "pathological" or "non-physiological" to each PPG pulse of a given unlabeled PPG signal.

In one aspect, time-related TF, normalized amplitude-related NAF and SNR-related features SNRF are determined for the PPG pulse being classified. The TF, NAF, SNRF are determined for the PPG pulse and are then inputted in the classifying machine learning model.

In an embodiment, the method comprises a step of training the classifying machine learning model by using expert-labelled data. The expert-labelled data are typically manually assigned labels by an expert based on an ECG signal. The expert-labelled data should not be confounded by the classes assigned to PPG pulses by classifying machine learning model.

As illustrated in **Figs. 5a to 5c****,** the step of training can comprise measuring an ECG signal (Fig. 5a) synchronously with the PPG signal (Fig. 5b). The cardiac contractions can be identified from the ECG signal (see black dots in Fig. 5a). The pulse upstrokes can be identified from the PPG signal (see black dots in Fig. 5b). Fig. 5c shows ECG-based RR intervals calculated from the ECG signal (dashed line in Fig. 5c) and PPG-based RR intervals calculated from the PPG signal (solid line in Fig. 5c).

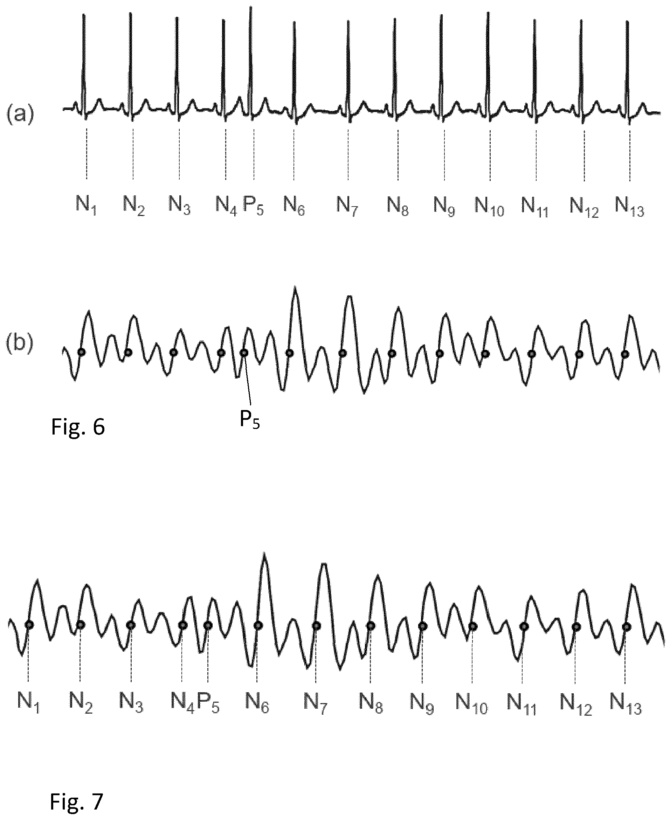

**Fig. 6a** shows an example of an ECG signal for which an expert (or a clinical device) has identified and classified each i^{th} cardiac cycle on the ECG signal as normal (Nᵢ) or pathological (Pᵢ). The ECG signal can be measured by using any ECG setup, e.g., a standard clinical 12-lead setup. The timing of the cardiac contractions is represented by the dots. In Fig. 6a, the expert has classified the fifth cardiac cycle as "pathological" (P₅). The other cardiac cycles are classified as "normal" (N₁ - N₄ and N₆ - N₁₃). The ECG signal of Fig. 6a does not comprise a "non-physiological" cardiac cycle.

**Fig. 6b** shows a PPG signal measured synchronously with the ECG signal. The timing of the pulse upstrokes, obtained by detecting the timing of systolic upstrokes in the PPG signal, are reported on the PPG signals with black dots. The expert classification from the ECG can be used to assign a label for each PPG pulse. Here, the fifth PPG pulse is labelled as "pathological" (P₅) while the other PPG pulses are labelled as "normal".

More generally, expert classification from the ECG signal can be used to assign a "non-physiological" label to PPG pulses which are not associated to a cardiac contraction. For instance, this situation can occur if a PPG pulse is mistakenly detected due to motion artefacts in the PPG signal. During a given period the number of detected pulse cycles can be higher than the number of detected cardiac cycles due to wrongly detected PPG pulses.

Moreover, a "non-physiological" label can be assigned to a PPG pulse that has low SNR characteristics. The "low SNR characteristics" is determined using the SNR-related features during the training of the classifying machine learning model (pulse classifier) using datasets including synchronous PPG and ECG signals (see below). Note that the SNR of a PPG pulse can be determined without requiring measuring an ECG signal.

The remaining non-labeled PPG pulses are either labelled as "normal" or "pathological" or are ignored.

By analyzing the shape of the ECG signal, an expert can identify normal cardiac contractions (originating from the sinoatrial node). Thus, the expert can assign the label "normal" to the PPG pulses that correspond to the ECG pulses analyzed as normal cardiac contractions.

By analyzing the shape of the ECG signal, an expert can identify abnormal, or pathological, cardiac contractions (not originating from the sinoatrial node). The expert can assign the label "pathological" to the PPG pulses that correspond to the ECG pulses analyzed as pathological.

In the case where the ECG signal comprises multiple cardiac contractions but only one single PPG pulse, no label is assigned to the PPG pulse (i.e. due to a missed detection of pulse upstrokes in the PPG or an extra detection of cardiac contractions in the ECG due to the presence of motion or other artifacts in the ECG signal). The PPG pulse is ignored in the training of the classifying machine learning algorithm.

Alternatively or in combination, the expert-labelled data can be obtained from an clinical device (for example a software) which automatically labels cardiac arrhythmia from ECG signals. In that case, the expression "expert" above can be read as "clinical device".

Table 1 shows the different labels applied to the PPG pulses based on the ECG signal expert analysis.

**Table 1**

| **Label** | **ECG** | **PPG** |
|---|---|---|
| Non-physiological (X) | Absence of cardiac cycle | Presence of one PPG pulse with high or low SNR characteristics |
| Non-physiological (X) | Presence of one cardiac cycle with normal or pathological cardiac contraction | Presence of one PPG pulse with low SNR characteristics |
| Normal (N) | Presence of one cardiac cycle with normal cardiac contraction (originating from the sinoatrial node) | Presence of one PPG pulse with high SNR characteristics |
| Pathological (P) | Presence of one cardiac cycle with pathological cardiac contraction (not originating from the sinoatrial node) | Presence of one PPG pulse with high SNR characteristics |
| *(TO BE IGNORED)* | Presence of more than one cardiac cycle | Presence of one single PPG pulse |

The step of training the classifying machine learning model (pulse classifier) by using expert-labelled data can comprise building a dataset including synchronous PPG and ECG signals together with a label attributed to each PPG pulse. The dataset may further include the time-related TF, normalized amplitude-related NAF and SNR-related SNRF features determined from each PPG pulse. The labels, possibly in combination with the time-related TF, normalized amplitude-related NAF and SNR-related SNRF features can be used to train the pulse classifier. The training can use supervised learning models such as support vector machines, decision trees, etc.

The resulting pulse classifier can then be used to classify each PPG pulse based on the time-related TF, normalized amplitude-related NAF and SNR-related SNRF features of the PPG pulse being classified. The pulse classifier further makes use of the statistical distribution of class features (TF, NAF, SNRF) of the preceding PPG pulses to provide an enhanced PPG pulse classification (to manage overlapping between N and P feature spaces).

The pulse classifier outputs a time series of pulse classes, whereby each PPG pulse is classified as "normal", "pathological" or "non-physiological". **Fig. 7** shows such a time series of pulse classes where one PPG pulse is classified as "pathological" (P₅, extrasystole) and the other PPG pulses are classified as "normal" (N₁ - N₄ and N₆ - N₁₃).

Conventional arrythmia detection can have strong limitations in terms of arrhythmia classifications. For example, conventional arrythmia detection based on PPG-based RR-intervals does not necessarily manage to separate AF episodes from sinus (normal) rhythm episodes with the presence of extrasystoles because both episodes will be characterized by large variations of RR interval values.

The time series of pulse classes, outputted from the pulse classifier, can be used to detect various cardiac arrhythmias and to improve the classification of cardiac arrhythmia.

### Enhanced AF detection

In an embodiment, the method comprises classifying AF. To that end, the classifications "normal" and "pathological" in the time series of classified PPG waveform can be represented as "0" for "normal" and "1" for "pathological". By doing so, one can obtain a time series of digitalized pulse classes (time series comprising the "0" and "1").

In conventional AF detection, a feature extraction algorithm processes RR intervals over time windows of a given duration (e.g. 30 seconds) to estimate features such as: 1) mean value of the RR intervals; 2) minimum value of the RR intervals; 3) maximum value of the RR intervals; 4) median value of the RR intervals and 5) interquartile range of the RR intervals.

The time series of digitalized pulse classes further allows for estimating additional features such as: 6) standard deviation of the digitalized pulse classes and 7) mean value of the digitalized pulse class values.

The set of features can be generated from known episodes of AF and sinus rhythm and can be used to train a classifier (e.g. a support vector machine) to separate set of features values related to AF from set of features values related to sinus rhythm. The trained classifier can be applied to any unknown portion of the PPG signal to detect the presence of AF.

In this example, features 6) and 7) allows for avoiding numerous episodes of false positives (e.g. episodes with extrasystoles).

### Enhanced cardiac arrhythmia classification

In another embodiment, the method comprises classifying cardiac arrhythmias by using the PPG pulses classified as "normal" and "pathological", and not the PPG pulses classified as "non-physiological". Cardiac arrhythmias may include AF, premature ventricular and atrial contractions, flutters, supraventricular and ventricular tachycardias as well as cardiac dysfunctions such as left branch block and AV node reentry.

In one aspect, the method uses the following set of features: time-related features and/or normalized amplitude-related features and/or the SNR-related features and/or the classifications "normal" and "pathological" in the time series of pulse classes. The set of features can be generated from known episodes of cardiac arrhythmias and can be used to train a classifier (e.g. a support vector machine) to separate set of features values related to specific cardiac arrhythmias. The trained classifier can be applied to any unknown portion of the PPG signal to classify cardiac rhythms.

Classifying cardiac arrhythmias can be performed for each PPG pulse individually (e.g. for single occurrences of extrasystoles) or for a succession of multiple pulses (e.g. for an episode of AF or trigeminy).

For example, AF episodes are characterized by the presence of pulses classified as "pathological" which appear randomly in between PPG pulses classified as "normal". In opposition, bigeminy, trigeminy episodes are characterized by a regular pattern of PPG pulses classified as "normal" and "pathological" (e.g. for bigeminy, the time series of pulse classes is characterized by a repetition of alternating N and P labels like Nₓ, Pₓ₊₁, Nₓ₊₂, Pₓ₊₃, Nₓ₊₄, ...). Classifying AF can thus comprise identifying random occurrence of the PPG pulses classified as "pathological" in the time series of pulse classes.

### Enhanced blood pressure (BP) and oxygen saturation (SpO₂) estimations

In another embodiment, the method comprises normalizing each PPG pulses to obtain normalized PPG pulses. The method further comprises averaging normalized PPG pulses classified as "normal" to obtain enhanced averaged normalized PPG pulses. The enhanced averaged normalized PPG pulses can then be used to estimate a blood pressure value or an SpO₂ value.

### Enhanced HRV feature extraction

In an embodiment, the method comprises determining HRV features by using the PPG pulses classified as "normal" and not the PPG pulses classified as "pathological" and "non-physiological".

The method uses the timing of a given periodic fiducial point in the PPG signal (e.g. pulse upstroke or pulse foot) associated to PPG pulses classified as "normal". The detected timings of the fiducial points classified as "non-physiological" and "pathological" are removed and replaced by interpolated detected timings of neighboring fiducial points classified as "normal". The resulting time series are denoted as enhanced PPG-based NN intervals which represent and indirect measure of the ECG-based NN intervals. In contrast to RR intervals, NN intervals exclude outliers such as ectopic beats and thus only include "normal" RR intervals.

From the ECG-based NN intervals, one can apply classic HRV feature formulas to extract time- and frequency-based features such as the SDNN (standard deviation of all NN intervals), SDANN (standard deviation of the averages of NN intervals in all segments of the entire recording), pNN50 (NN50 count divided by the total number of all NN intervals), VLF (power in the very low frequency range ≤ 0.04 Hz of NN intervals), LF (power in low frequency range (0.04 < *f* ≤ 0.15 Hz) of NN intervals) and HF (power in high frequency range (0.15 < *f* ≤ 0.4 Hz) of NN intervals). The same or any other suitable technique can be applied to the enhanced PPG-based NN intervals leading to enhanced time-based features and enhanced frequency-based HRV features.

### Other HRV-based measurements

In another embodiment, the method comprises determining enhanced time-based features and enhanced frequency-based HRV features from the enhanced PPG-based NN intervals. The method further comprises determining any one of stress level, sleep stages, fatigue/recovery, respiration, circadian cycle, sleep apnoea or other sleep disorders by using the determining enhanced time- and enhanced frequency-based HRV features.

## Claims

1. A method for classifying photoplethysmography (PPG) pulses, comprising:
measuring a PPG signal during a measurement time period such as to obtain a time series of PPG pulses; and
during said measurement time period, identifying individual PPG pulses in the PPG signal, each PPG pulse corresponding to a pulse cycle;
for each PPG pulse, using a pulse-wave analysis technique to determine, within the pulse cycle, at least a time-related feature comprising a time duration, and/or a normalized amplitude-related pulse-related feature and/or a SNR-related pulse-related feature;
**characterized in that**
for each PPG pulse, using a machine learning model in combination with at least one of the determined features: a time-related, and/or a normalized amplitude-related and/or a SNR-related feature for the PPG pulse, to classify each PPG pulse in the pre-processed PPG signal as "normal", "pathological" or "non-physiological" such as to output a time series of pulse classes.

2. The method according to claim 1,
comprising a step of training the machine learning model by using expert-labelled data.

3. The method according to claim 2,
wherein the expert-labelled data are assigned by an expert based on an ECG signal and/or obtained from a clinical device which automatically labels cardiac arrhythmia from an ECG signal.

4. The method according to claim 2 or 3,
comprising measuring an ECG signal synchronously with the PPG signal and identifying cardiac contractions from the measured ECG signal;
wherein expert-labelled data comprise labelling PPG pulses which are not associated to a cardiac contraction as "non-physiological"; and
wherein expert-labelled data further comprise labeling PPG pulses which are associated to normal cardiac contraction as "normal", and
labeling PPG pulses which are associated to pathological cardiac contraction as "pathological"..

5. The method according to claim(s) 1 to 4,
wherein expert-labelled data further comprise labeling PPG pulses which are associated to low SNR characteristics as "non-physiological.

6. The method according to any one of claims 1 to 5,
comprising classifying cardiac arrhythmias by using the PPG pulses classified as "normal" and "pathological", and not the PPG pulses classified as "non-physiological".

7. The method according to claim 6,
wherein classifying cardiac arrhythmias further comprise using at least one of: a time-related feature comprising a time duration, and/or a normalized amplitude-related pulse-related feature and/or a SNR-related pulse-related feature.

8. The method according to claim 6 or 7,
wherein classifying cardiac arrhythmias include atrial fibrillation (AF), extrasystoles and tachycardia, premature ventricular and atrial contractions, flutters, supraventricular and ventricular tachycardias as well as cardiac dysfunctions such as left branch block and AV node reentry.

9. The method according to any one of claims 1 to 5,
comprising detecting atrial fibrillation (AF) by identifying random occurrence of the "pathological" pulse class in the time series of pulse classes.

10. The method according to claim 9,
wherein detecting AF comprises using at least one of: a time-related feature comprising a time duration, and/or a normalized amplitude-related pulse-related feature and/or a SNR-related pulse-related feature.

11. The method according to any one of claims 1 to 5,
further comprising normalizing each PPG pulses to obtain normalized PPG pulses;
averaging normalized PPG pulses classified as "normal" to obtain enhanced averaged normalized PPG pulses; and
using the enhanced averaged normalized PPG pulses to estimate a blood pressure value or a SpO₂ value.

12. The method according to any one of claims 1 to 5,
comprising determining HRV features by using the time series of "normal" pulses classes and not the PPG pulses classified as "pathological" and "non-physiological".

13. The method according to claim 12,
comprising identifying a timing of each PPG pulse classified as "normal" in the time series of pulse classes, wherein timings of "non-physiological" and "pathological" pulse classes are replaced by interpolated timings of nearest "normal" PPG pulses resulting in enhanced PPG-based NN intervals; and comprising determining enhanced time-based features and enhanced frequency-based HRV features from the timing of the enhanced PPG-based NN intervals.

14. The method according to claim 13,
comprising determining any one of stress level, sleep stages, fatigue/recovery, respiration, circadian cycle, sleep apnoea or other sleep disorders by using the determined enhanced time- and enhanced frequency-based HRV features.

15. The method according to any one of claims 1 to 14,
wherein said identifying individual PPG pulses comprises detecting fiducial points in the PPG signal.

16. The method according to any one of claims 1 to 15, comprising removing low frequency components of the PPG signal.

17. The method according to claim 16,
comprising removing a baseline wander of the PPG signal.
